(19) Europäisches Patentamt
European Patent Office
Office européen des brevets

(11) **EP 4 622 396 A1**

(12) **EUROPEAN PATENT APPLICATION**

(43) Date of publication:
**24.09.2025 Bulletin 2025/39**

(21) Application number: **25159597.1**

(22) Date of filing: **24.02.2025**

(51) International Patent Classification (IPC):
**H05G 1/10** (2006.01) **H05G 1/54** (2006.01)
**H05G 1/22** (2006.01)

(52) Cooperative Patent Classification (CPC):
**H05G 1/10; H05G 1/54;** H05G 1/22

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **19.03.2024 IN 202441020353**

(71) Applicant: **GE Precision Healthcare LLC
Waukesha, WI 53188 (US)**

(72) Inventors:
• **LARROUX, Jean François**
**78533 Buc (FR)**
• **LOUVRIER, Yannick**
**78533 Buc (FR)**
• **LEVILLY, Nicolas**
**78533 Buc (FR)**
• **VOREM, Sravan**
**560066 Bengaluru (IN)**

(74) Representative: **AWA Sweden AB
Box 45086
104 30 Stockholm (SE)**

(54) **X-RAY TUBE FILAMENT AND FILAMENT DRIVER CIRCUIT FAILURE ISOLATION**

(57) Methods and systems are provided for detecting and identifying a degraded component of a filament drive circuit (500) of an X-ray imaging system (100, 200). In one example, the degraded component may be identified by including diagnostic capacitors (502, 504) at various locations in the filament drive circuit (500), which may alter a resonant frequency of the filament drive circuit (500) in the event of a degraded filament (414), cable (413), or different component of the filament drive circuit (500). During a diagnostic procedure, a voltage pulse may be performed on the filament drive circuit (902), and a resulting current may be measured and converted into a digital signal (904). The digital signal may be compared to a set of reference resonant frequencies stored in a lookup table in a memory of the X-ray imaging system (908), where a matching resonant frequency may indicate which component of the filament drive circuit (500) is degraded.

FIG. 5

**Description**

CROSS REFERENCE TO RELATED APPLICATIONS

The present application claims priority to Indian Patent Application No. 202441020353, entitled "X-RAY TUBES FILAMENT FAILURE REMOTE ISOLATION", and filed on March 19, 2024. The entire contents of the above-listed application is hereby incorporated by reference for all purposes.

TECHNICAL FIELD

Embodiments of the subject matter disclosed herein relate to medical imaging systems, and in particular, to diagnosing degradation conditions of an X-ray tube.

BACKGROUND

In X-ray based imaging systems, such as computed tomography (CT) imaging systems, an electron beam generated by a cathode is directed towards a target within an X-ray tube. In some embodiments, the target may be an anode, while in other embodiments, the X-ray tube may include an anode separate from the target. A fan-shaped or cone-shaped beam of X-rays produced by electrons colliding with the target is directed towards an object, such as a patient. After being attenuated by the object, the X-rays impinge upon an array of radiation detectors, generating an image.
The cathode may comprise a filament, where the beam of X-rays may be generated by introducing an electric current into the filament via a filament drive circuit. The filament drive circuit may include various components, where a degradation in one of the components may cause a short circuit that renders the X-ray tube inoperable. However, it may be difficult to discriminate between a first degradation condition (e.g., a failure) of a first component of the filament drive circuit (e.g., an opened filament), and a second degradation condition of a second component of the filament drive circuit (e.g., a high-voltage transformer). As a result, when making repairs, field engineers may order parts to repair various components, leading to unnecessary expenditures and increased time spent on repairs.
One approach to addressing this problem is to include a plurality of switches in the filament drive circuit that may be opened or closed to isolate portions of the filament drive circuit. A diagnostic procedure may then be performed that checks the portions of the filament drive circuit individually. However, the switches may increase a cost of the filament drive circuit and a complexity of the operation and control of the filament drive circuit. Further, the addition of the switches and high-voltage insulation used for the switches may increase a size of the X-ray tube, which may entail costly adjustments of a design of an imaging system.

SUMMARY

The current disclosure at least partially addresses one or more of the above identified issues by a system, comprising an x-ray tube including a filament, a high-voltage connector, and a first diagnostic capacitor in the high-voltage connector; and a filament drive circuit configured to supply current to the filament of the x-ray tube, the filament drive circuit including an inverter and a second diagnostic capacitor.
The above advantages and other advantages, and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

BRIEF DESCRIPTION OF THE DRAWINGS

Various aspects of this disclosure may be better understood upon reading the following detailed description and upon reference to the drawings in which:

FIG. 1 shows a pictorial view of an imaging system, in accordance with one or more embodiments of the present disclosure;
FIG. 2 shows a block schematic diagram of an exemplary imaging system, in accordance with one or more embodiments of the present disclosure;
FIG. 3 is a schematic diagram of an exemplary X-ray tube, in accordance with one or more embodiments of the present disclosure;

FIG. 4 shows a first filament drive circuit including an X-ray tube, as prior art;

FIG. 5 shows a second filament drive circuit including an X-ray tube and diagnostic capacitors, in accordance with one or more embodiments of the present disclosure;

FIG. 6 shows the second filament drive circuit with an open X-ray tube filament, in accordance with one or more embodiments of the present disclosure;

FIG. 7 shows the second filament drive circuit with an open cable, in accordance with one or more embodiments of the present disclosure;

FIG. 8 shows a graph illustrating different resonant frequencies of a filament resonant tank, in accordance with one or more embodiments of the present disclosure;

FIG. 9 is a flowchart illustrating an exemplary method for determining a degraded component of a filament drive circuit, in accordance with one or more embodiments of the present disclosure; and

FIG. 10 is a graph illustrating an exemplary resonant frequency of a filament resonant tank when a voltage pulse is applied, in accordance with one or more embodiments of the present disclosure.

The drawings illustrate specific aspects of the described systems and methods. Together with the following description, the drawings demonstrate and explain the structures, methods, and principles described herein. In the drawings, the size of components may be exaggerated or otherwise modified for clarity. Well-known structures, materials, or operations are not shown or described in detail to avoid obscuring aspects of the described components, systems and methods.

DETAILED DESCRIPTION

This description and embodiments of the subject matter disclosed herein relate to methods and systems for a medical imaging system including an X-ray source. While a computed tomography imaging (CT) system is described herein, it should be appreciated that the systems and methods described herein may be used with other types of X-ray imaging systems without departing from the scope of this disclosure. Typically, the X-ray source emits a fan-shaped beam or a cone-shaped beam towards an object, such as a patient. In some X-ray imaging systems, such as in CT systems, the X-ray source and the detector array are rotated about a gantry within an imaging plane and around the patient, and images are generated from projection data at a plurality of views at different view angles. In other X-ray imaging systems, the X-ray source and the detector array may have a fixed position.

The beam, after being attenuated by the patient, impinges upon an array of radiation detectors. The X-ray detector or detector array typically includes a collimator for collimating X-ray beams received at the detector, a scintillator disposed adjacent to the collimator for converting X-rays to light energy, and photodiodes for receiving the light energy from the adjacent scintillator and producing electrical signals therefrom. An intensity of the attenuated beam radiation received at the detector array is typically dependent upon the attenuation of the X-ray beam by the patient. Each detector element of a detector array produces a separate electrical signal indicative of the attenuated beam received by each detector element. The electrical signals are transmitted to a data processing system for analysis. The data processing system processes the electrical signals to facilitate generation of an image.

The X-ray source may include an X-ray tube that may be realized as a vacuum tube diode including a cathode and an anode. An inside of the X-ray tube may be set as a high vacuum state of about 10 mmHg. The cathode may comprise a filament that is heated to a high temperature to generate thermal electrons, by applying a current (e.g., the tube current) to an electric wire connected to the filament. A voltage difference (e.g., the tube voltage) may then be applied between the cathode and the anode, which causes the thermal electrons to accelerate towards and collide with the anode, generating an X-ray. This beam of electrons may be focused via electrostatic controls, electromagnetic controls, or a combination of electrostatic and electromagnetic controls. X-rays emitted as a result of the electrons colliding with the target are focused on the patient. When the tube voltage increases, a velocity of the thermal electrons increases, and accordingly, an energy of the X-ray (that is, energy of the photons) that is generated when the thermal electrons collide with the target is increased. When the tube current increases, the number of thermal electrons emitted from the filament is increased, and accordingly, the X-ray dose (that is, the number of X-ray photons) generated when the thermal electrons collide with the target material is increased. Thus, the energy of the X-ray may be adjusted according to the tube voltage, and the intensity of the X-ray or the X-ray dose may be adjusted according to the tube current and the X-ray exposure time.

The tube current may be generated by a filament drive circuit that electrically couples the filament with a DC voltage supply. The filament drive circuit may include various components for switching on, controlling, and transmitting the current to the filament, such as one or more switches, one or more capacitors, a filament resonant tank, a high-voltage transformer, and various low-voltage and high-voltage wires. A degradation in any of the components could result in the failure of the X-ray tube. For example, the failure of the X-ray tube may be due to an opened filament (e.g., due to an evaporation of tungsten during a lifetime of the X-ray tube), or a degradation of the high-voltage transformer, or a short circuit in one of the high-voltage wires, or a degradation of a different component.

However, In X-ray systems it may be difficult to discriminate between X-ray tube failures due to an opened filament, and other failures in the filament drive circuit. Embedded voltage and/or current sensors may not be sufficient to isolate a degradation, and diagnosing the degradation may rely on checking an impedance at various locations in the filament drive circuit with a multimeter, which may be time consuming and burdensome. An engineer may go onsite, stop the X-ray system and wait for the X-ray system to cool down, measure the impedance at the various locations, open the cable, etc. As a result, when making repairs, field engineers may order parts to repair various components rather than pursuing an accurate diagnosis, leading to unnecessary expenditures and increased time spent on repairs. For example, the field engineer may order a new filament, a new generator, and a new cable, rather than opening up the X-ray tube to identify the degraded component prior to making the order to facilitate a quicker repair. Some components, like the generator, may be more costly to repair than others, like the filament or cable, and identifying a degradation in a less expensive component prior to ordering replacement parts may reduce costs.

Diagnosis of a failed X-ray tube may be facilitated by including additional switches in the filament drive circuit that may be opened or closed to isolate portions of the filament drive circuit, such that the portions of the filament drive circuit may be individually tested for degraded components. However, the switches may increase a cost and size of the filament drive circuit, and a complexity of control and diagnostic procedures used during operation of the X-ray tube.

To address this problem, systems and methods are described below to adjust a design of the filament drive circuit to include additional capacitors at strategic locations within the filament drive circuit. The capacitors have no effect on the behavior of the filament drive circuit during operation of the X-ray tube. However, the capacitors may change a resonant frequency of the filament drive circuit depending on a location of a degraded component of the filament drive circuit. In other words, as a result of the inclusion of the capacitors, an open filament may result in a first resonant frequency, and an open cable may result in a second, different resonant frequency. Thus, a diagnostic procedure may measure the resonant frequency, and compare the resonant frequency with a set of reference frequencies stored in a lookup table to identify the degraded component. In this way, a quick and efficient method for diagnosing a failed X-ray tube and detecting degraded components of the filament drive circuit is provided, that does not rely on additional switches. By identifying the degraded component in this way, a field engineer may order specific parts to repair the filament drive circuit, reducing a service cost of the X-ray tube and a repair time.

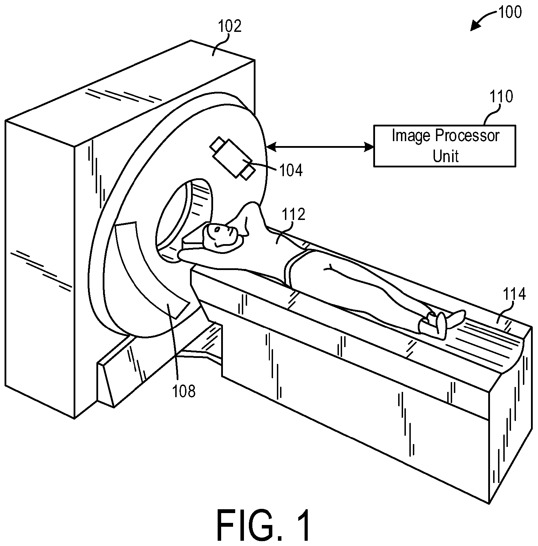

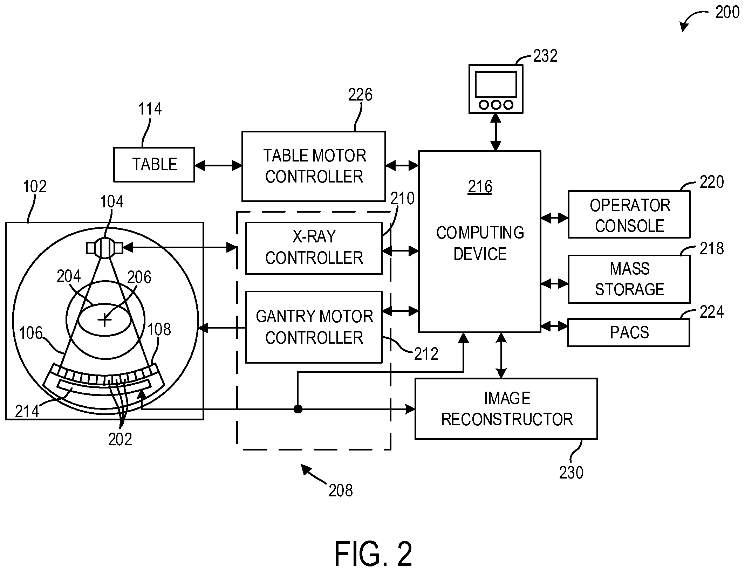

FIG. 1 illustrates an exemplary X-ray system 100 configured for CT imaging. It should be appreciated that in other embodiments, X-ray system 100 may be a different type of X-ray imaging system. The X-ray system 100 is configured to image a subject 112 such as a patient, an inanimate object, one or more manufactured parts, and/or foreign objects such as dental implants, stents, and/or contrast agents present within the body. In one embodiment, the X-ray system 100 includes a gantry 102, which in turn, may further include at least one X-ray source 104 configured to project a beam of X-ray radiation 106 (see FIG. 2) for use in imaging the subject 112 laying on a table 114. Specifically, the X-ray source 104 is configured to project the X-ray radiation beams 106 towards a detector array 108 positioned on the opposite side of the gantry 102. Although FIG. 1 depicts a single X-ray source 104, in certain embodiments, multiple X-ray sources and detectors may be employed to project a plurality of X-ray radiation beams for acquiring projection data at different energy levels corresponding to the patient. In some embodiments, the X-ray source 104 may enable dual-energy gemstone spectral imaging (GSI) by rapid peak kilovoltage (kVp) switching. In some embodiments, the X-ray detector employed is a photon-counting detector which is capable of differentiating X-ray photons of different energies. In other embodiments, two sets of X-ray sources and detectors are used to generate dual-energy projections, with one set at low-kVp and the other at high-kVp. It should thus be appreciated that the methods described herein may be implemented with single energy acquisition techniques as well as dual energy acquisition techniques.

In certain embodiments, the X-ray system 100 further includes an image processor unit 110 configured to reconstruct images of a target volume of the subject 112 using an iterative or analytic image reconstruction method. For example, the image processor unit 110 may use an analytic image reconstruction approach such as filtered back projection (FBP) to reconstruct images of a target volume of the patient. As another example, the image processor unit 110 may use an iterative image reconstruction approach such as advanced statistical iterative reconstruction (ASIR), conjugate gradient (CG), maximum likelihood expectation maximization (MLEM), model-based iterative reconstruction (MBIR), and so on to reconstruct images of a target volume of the subject 112. As described further herein, in some examples the image processor unit 110 may use both an analytic image reconstruction approach such as FBP in addition to an iterative image reconstruction approach.

In some CT imaging system configurations, an X-ray source projects a cone-shaped X-ray radiation beam which is collimated to lie within an X-Y-Z plane of a Cartesian coordinate system and generally referred to as an "imaging plane." The X-ray radiation beam passes through an object being imaged, such as the patient or subject. The X-ray radiation beam, after being attenuated by the object, impinges upon an array of detector elements. The intensity of the attenuated X-ray radiation beam received at the detector array is dependent upon the attenuation of an X-ray radiation beam by the object. Each detector element of the array produces a separate electrical signal that is a measurement of the X-ray beam attenuation at the detector location. The attenuation measurements from all the detector elements are acquired separately to produce a transmission profile.

In some CT systems, the X-ray source and the detector array are rotated with a gantry within the imaging plane and around the object to be imaged such that an angle at which the X-ray beam intersects the object constantly changes. A group of X-ray radiation attenuation measurements, e.g., projection data, from the detector array at one gantry angle is referred to as a "view." A "scan" of the object includes a set of views made at different gantry angles, or view angles, during one revolution of the X-ray source and detector.

The X-ray source 104 includes an anode and a cathode. Electrons emitted by the cathode (e.g., resulting from energization of the cathode) may be intercepted by a target arranged at or near the anode. Electrons intercepted by the target may release energy in the form of X-rays, with the X-rays being directed toward the detector array 108. An area of the target surface that receives the electrons from the cathode and forms the emitted X-rays may be referred to herein as a focal spot. The emitted X-rays may be focused on a portion of the scanned subject 204, at an effective focal spot.

FIG. 2 illustrates an exemplary X-ray imaging system 200 similar to the X-ray system 100 of FIG. 1. In accordance with aspects of the present disclosure, the X-ray imaging system 200 is configured for imaging a subject 204 (e.g., the subject 112 of FIG. 1). In one embodiment, the X-ray imaging system 200 includes the detector array 108 (see FIG. 1). The detector array 108 further includes a plurality of detector elements 202 that together sense the X-ray radiation beam 106 (see FIG. 2) that pass through the subject 204 (such as a patient) to acquire corresponding projection data. In some embodiments, the detector array 108 may be fabricated in a multi-slice configuration including the plurality of rows of cells or detector elements 202, where one or more additional rows of the detector elements 202 are arranged in a parallel configuration for acquiring the projection data.

In certain embodiments, the X-ray imaging system 200 is configured to traverse different angular positions around the subject 204 for acquiring desired projection data. Accordingly, the gantry 102 and the components mounted thereon may be configured to rotate about a center of rotation 206 for acquiring the projection data, for example, at different energy levels. Alternatively, in embodiments where a projection angle relative to the subject 204 varies as a function of time, the mounted components may be configured to move along a general curve rather than along a segment of a circle.

As the X-ray source 104 and the detector array 108 rotate, the detector array 108 collects data of the attenuated X-ray beams. The data collected by the detector array 108 undergoes pre-processing and calibration to condition the data to represent the line integrals of the attenuation coefficients of the scanned subject 204. The processed data are commonly called projections. In some examples, the individual detectors or detector elements 202 of the detector array 108 may include photon-counting detectors which register the interactions of individual photons into one or more energy bins. It should be appreciated that the methods described herein may also be implemented with energy-integrating detectors.

In one embodiment, the X-ray imaging system 200 includes a control mechanism 208 to control movement of the components such as rotation of the gantry 102 and the operation of the X-ray source 104. In certain embodiments, the control mechanism 208 further includes an X-ray controller 210 configured to provide power and timing signals to the X-ray source 104. Additionally, the control mechanism 208 includes a gantry motor controller 212 configured to control a rotational speed and/or position of the gantry 102 based on imaging requirements.

In certain embodiments, the control mechanism 208 further includes a data acquisition system (DAS) 214 configured to sample analog data received from the detector elements 202 and convert the analog data to digital signals for subsequent processing. The DAS 214 may be further configured to selectively aggregate analog data from a subset of the detector elements 202 into so-called macro-detectors, as described further herein. The data sampled and digitized by the DAS 214 is transmitted to a computer or computing device 216. In one example, the computing device 216 stores the data in a storage device or mass storage device 218. The storage device 218, for example, may be any type of non-transitory memory and may include a hard disk drive, a floppy disk drive, a compact disk-read/write (CD-R/W) drive, a Digital Versatile Disc (DVD) drive, a flash drive, and/or a solid-state storage drive.

Additionally, the computing device 216 provides commands and parameters to one or more of the DAS 214, the X-ray controller 210, and the gantry motor controller 212 for controlling system operations such as data acquisition and/or processing. In certain embodiments, the computing device 216 controls system operations based on operator input. The computing device 216 receives the operator input, for example, including commands and/or scanning parameters via an operator console 220 operatively coupled to the computing device 216. The operator console 220 may include a keyboard (not shown) or a touchscreen to allow the operator to specify the commands and/or scanning parameters.

Although FIG. 2 illustrates one operator console 220, more than one operator console may be coupled to the X-ray imaging system 200, for example, for inputting or outputting system parameters, requesting examinations, plotting data, and/or viewing images. Further, in certain embodiments, the X-ray imaging system 200 may be coupled to multiple displays, printers, workstations, and/or similar devices located either locally or remotely, for example, within an institution or hospital, or in an entirely different location via one or more configurable wired and/or wireless networks such as the Internet and/or virtual private networks, wireless telephone networks, wireless local area networks, wired local area networks, wireless wide area networks, wired wide area networks, etc.

In one embodiment, for example, the X-ray imaging system 200 either includes, or is coupled to, a picture archiving and communications system (PACS) 224. In an exemplary implementation, the PACS 224 is further coupled to a remote system such as a radiology department information system, hospital information system, and/or to an internal or external network (not shown) to allow operators at different locations to supply commands and parameters and/or gain access to the image data.

The computing device 216 uses the operator-supplied and/or system-defined commands and parameters to operate a table motor controller 226, which in turn, may control a table 114 which may be a motorized table. Specifically, the table motor controller 226 may move the table 114 for appropriately positioning the subject 204 in the gantry 102 for acquiring projection data corresponding to the target volume of the subject 204.

As previously noted, the DAS 214 samples and digitizes the projection data acquired by the detector elements 202. Subsequently, an image reconstructor 230 uses the sampled and digitized X-ray data to perform high-speed reconstruction. Although FIG. 2 illustrates the image reconstructor 230 as a separate entity, in certain embodiments, the image reconstructor 230 may form part of the computing device 216. Alternatively, the image reconstructor 230 may be absent from the X-ray imaging system 200 and instead the computing device 216 may perform one or more functions of the image reconstructor 230. Moreover, the image reconstructor 230 may be located locally or remotely, and may be operatively connected to the X-ray imaging system 200 using a wired or wireless network. Particularly, one exemplary embodiment may use computing resources in a "cloud" network cluster for the image reconstructor 230.

In one embodiment, the image reconstructor 230 stores the images reconstructed in the storage device 218. Alternatively, the image reconstructor 230 may transmit the reconstructed images to the computing device 216 for generating useful patient information for diagnosis and evaluation. In certain embodiments, the computing device 216 may transmit the reconstructed images and/or the patient information to a display or display device 232 communicatively coupled to the computing device 216 and/or the image reconstructor 230. In some embodiments, the reconstructed images may be transmitted from the computing device 216 or the image reconstructor 230 to the storage device 218 for short-term or long-term storage.

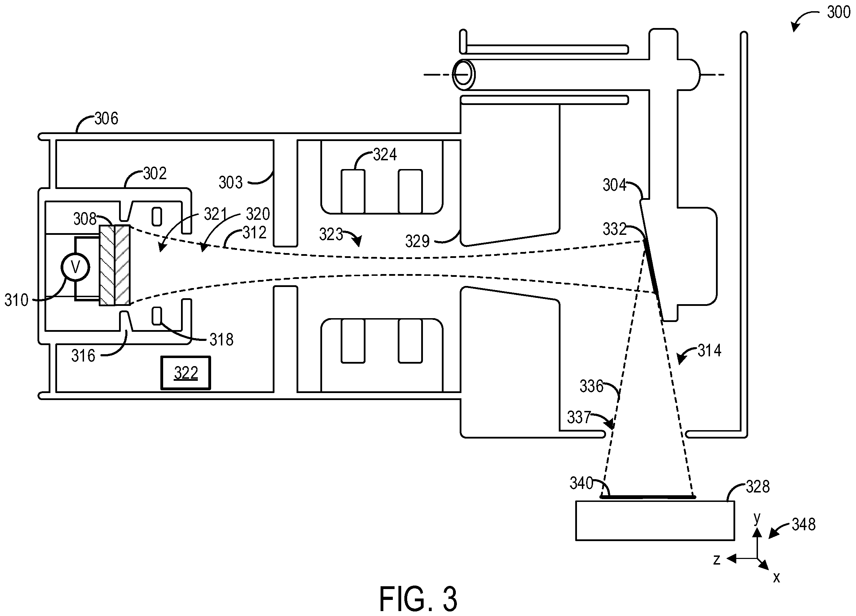

Referring now to FIG. 3, an exemplary X-ray tube 300 is shown. In one embodiment, the X-ray tube 300 may be the X-ray source 104 of FIGS. 1-2. In the illustrated embodiment, the X-ray tube 300 includes an exemplary cathode 302 and an anode 303 disposed within a tube casing 306. The cathode may include a filament 308. The cathode 302, and in particular the filament 308, may be directly heated by passing a current through the filament 308, which may be supplied by a voltage source 310. In one embodiment, a current of about 10 amps (A) may be passed through the filament 308. The filament 308 may emit an electron beam 312 as a result of being heated by the current supplied by the voltage source 310. As used herein, the term "electron beam" may be used to refer to a stream of electrons that have substantially similar velocities.

The electron beam 312 may be directed towards a target 304 to produce X-rays 314. More particularly, the electron beam 312 may be accelerated from the filament 308 towards the target 304 by applying a potential difference between the filament 308 and the anode 303. In one embodiment, a high-voltage in a range from about 40 kV to about 450 kV may be applied to set up the potential difference between the filament 308 and the anode 303, thereby generating one or more electric fields 320 in the X-ray tube 300. In one embodiment, a high-voltage differential of about 140 kV may be applied between the filament 308 and the anode 303 to accelerate the electrons in the electron beam 312 towards the target 304. As an example, the filament 308 may be at a potential of about -140 kV and the anode 303 and target 304 may be at ground potential or about zero volts.

The electron beam 312 may impinge on the target 304 at a focal spot 332. When the electron beam 312 impinges upon the target 304, heat may be generated in the target 304 at a location of the focal spot 332, which may be significant enough to melt the target 304. In various embodiments, a rotating target may be used to circumvent the problem of heat generation in the target 304. For example, the target 304 may be configured to rotate such that the focal spot 332 generated by the electron beam 312 striking the target 304 does not strike the target 304 consistently at the same location, so that the target 304 may not melt. In various embodiments, the target 304 may include materials such as, but not limited to, tungsten or molybdenum.

The size of a focal spot on the target 304 may also be adjusted to reduce an amount of heat generated in the target 304, where a smaller focal spot may generate a greater amount of heat at a specific location. An electron collector 329, held at a same potential as the target 304, serves as a sink of electrons that bounce off the surface of 304 during the initial impact, which reduces the chance of those same electrons re-striking the target. Collecting the backscattered electrons in this way further may reduce target heating.

The X-ray tube 300 may include one or more focusing electrodes 316, which may be disposed adjacent to the filament 308 such that the one or more focusing electrodes 316 focus the electron beam 312 towards the target 304. As used herein, the term "adjacent" means near to in space or position. To focus the electron beam 312, voltages may be applied to the one or more focusing electrodes 316 to generate the one or more electric fields 321. The voltages may be different for each of the one or more focusing electrodes 316. For example, a first voltage may be applied to a first focusing electrode 316; a second voltage may be applied to a second focusing electrode 316; a third voltage may be applied to a third focusing electrode 316; and so on. For some focusing electrodes 316, the voltage may be 0, where no voltage is applied to the focusing electrode 316. In some embodiments, a first portion of the focusing electrodes 316 may be used for deflecting the electron beam 312, and a second portion of the focusing electrodes 316 may be used for focusing the electron beam 312. In this way, the voltages may be selectively applied by a controller of a control electronics module 322 to generate one or more specific electric fields that focus the electron beam 312 to a desired shape and deflect the electron beam 312 to a desired position.

In some embodiments, the one or more focusing electrodes 316 may each be maintained at a voltage potential that is less than a voltage potential of the filament 308. The potential difference between the filament 308 and the one or more focusing electrodes 316 may prevent electrons generated from the filament 308 from moving towards the one or more focusing electrodes 316. In some embodiments, the one or more focusing electrodes 316 may be maintained at a negative potential with respect to that of the filament 308. The negative potential of the one or more focusing electrodes 316 with respect to the filament 308 may focus the electron beam 312 away from the one or more focusing electrodes 316, thereby facilitating focusing of the electron beam 312 towards the target 304.

In other embodiments, the one or more focusing electrodes 316 may be maintained at a voltage potential that is equal to or substantially similar to the voltage potential of the filament 308. The similar voltage potential of the one or more focusing electrodes 316 with respect to the voltage potential of the filament 308 may create a parallel electron beam by shaping electrostatic fields due to the shape of the one or more focusing electrodes 316. The one or more focusing electrodes 316 may be maintained at a voltage potential that is equal to or substantially similar to the voltage potential of the filament 308 via use of a lead coupling the filament 308 and the one or more focusing electrodes 316.

Additionally, the X-ray tube 300 may include one or more extraction electrodes 318, which may be used for additionally controlling and focusing the electron beam 312 towards the anode 303. The one or more extraction electrodes 318 may be located between the anode 303 and the filament 308. In some embodiments, the one or more extraction electrodes 318 may be positively biased by supplying a desired voltage to the one or more extraction electrodes 318.

An energy of the electron beam 312 may be controlled in various ways. For instance, the energy the electron beam 312 may be controlled by altering the potential difference (e.g., an acceleration voltage) between the cathode 302 and the anode 303. As used herein, the term "electron beam current" refers to a flow of electrons per second between the cathode 302 and the anode 303. The current of the electron beam 312 may be controlled by adjusting the filament voltage to change the temperature of the filament 308. The electron beam current may be controlled by altering the voltage applied to the one or more extraction electrodes 318. It may be noted that the filament 308 may be treated as an infinite source of electrons.

The one or more electric fields 321 may be generated between the one or more extraction electrodes 318 and the one or more focusing electrodes 316 due to a potential difference between the one or more focusing electrodes 316 and the one or more extraction electrodes 318. A strength of the one or more electric fields 320 may be employed to control the intensity of electron beam 312 generated by the filament 308 towards the anode 303. More particularly, the one or more electric fields 320 may cause the electrons emitted by the filament 308 to be accelerated towards the anode 303. The stronger the one or more electric fields 320, the stronger the acceleration of the electrons from the filament 308 towards the anode 303. Alternatively, the weaker the one or more electric fields 320, the lesser the acceleration of electrons from the filament 308 towards the anode 303. The intensity of the electron beam 312 striking the target 304 may thus be controlled by the one or more electric fields 320 and 321.

Furthermore, voltage shifts of 8 kV or less may be applied to the one or more extraction electrodes 318 to control the intensity of the electron beam 312. In certain embodiments, these voltage shifts may be applied to the one or more extraction electrodes 318 via use of the control electronics module 322. The control electronics module 322 may be a non-limiting embodiment of, or may be a part of X-ray controller 210 of FIG. 2.

Additionally, the X-ray tube 300 may also include a one or more magnets 324 for focusing and/or positioning and deflecting the electron beam 312 onto the target 304. In various embodiments, the one or more magnets 324 may be disposed between the cathode 302 and the target 304. In some embodiments, the one or more magnets 324 may include one or more multipole magnets for influencing focusing of the electron beam 312 by creating one or more magnetic fields 323 that shapes the electron beam 312 on the target 304. The one or more multipole magnets may include one or more quadrupole magnets, one or more dipole magnets, or combinations thereof.

As properties of the electron beam current and voltage change, electrostatic focusing of the electron beam 312 will change accordingly. In order to maintain a stable size, shape, and other characteristics of a focal spot, or quickly modify focal spot size and/or shape according to system requirements, the one or more magnets 324 may provide a magnetic field having a performance controllable from steady-state to a sub-30 microsecond time scale for a wide range of focal spot sizes and shapes. When the electron beam 312 has been focused and positioned, the electron beam 312 impinges upon the target 304 at a focal spot 332 to generate the X-rays 314. The X-rays 314 generated by collision of the electron beam 312 with the target 304 may be directed from the X-ray tube 300 through an opening in the tube casing 306, at an X-ray window 337, towards an object 328.

As a result of the electron beam 312 colliding with target 304 at the focal spot 332, a set of X-rays 336 may be generated and directed out X-ray window 337 towards the object 328. The set of X-rays 336 may intersect with the object 328 at an effective focal spot 340. The effective focal spot may have a width (in an X dimension, as indicated by coordinate axes 348) and a length (in an Z dimension, as indicated by the coordinate axes 348).

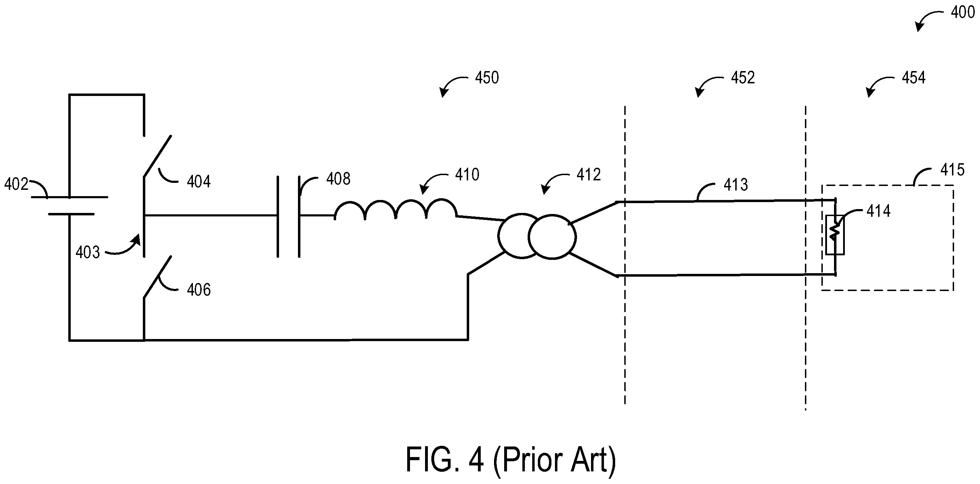

Referring now to FIG. 4, an exemplary filament drive circuit 400 is shown as prior art, where the filament drive circuit 400 may be used to heat a cathode of an X-ray tube of an X-ray imaging system, such as X-ray imaging systems 100 and 200, via an electric current. The cathode, X-ray tube, and electric current may be non-limiting examples of the cathode 302, the X-ray tube 300, and the electron beam current of FIG. 3.

The filament drive circuit 400 may include a generator portion 450, a cable portion 452, and an X-ray tube portion 454. The generator portion 450 includes a voltage source 402, which may apply a first voltage to generate a first electric current. For example, a first voltage of about 10V may be applied to generate a first electric current of about 3 to 5A. The generator portion 450 further comprises an inverter 403 in full-bridge or half bridge topology, and a high-voltage transformer 412. The inverter 403 supplies an inductor 410 that may optimize a leakage inductance of the high-voltage transformer 412. The high-voltage transformer 412 may transform the first voltage into a second, higher voltage. For example, the second, higher voltage may be up to a crest value of 300 kVp.

The X-ray tube portion 454 includes a filament 414 (e.g., the cathode) of an X-ray tube assembly 415, which is heated by a second, higher electric current generated from the second, higher voltage. In one example, the filament 414 is a tungsten filament. The cable portion 452 includes a high-voltage cable 413, where the second, higher electric current is transmitted to the filament 414 via the high-voltage cable 413. The second, higher current generates sufficient heat for electrons at the filament 414 to be accelerated to collide with a target of an anode of the X-ray tube assembly 415 (not shown in FIG. 4), to generate X-rays.

The filament drive circuit 400 may include a first switch 404 and a second switch 406, and a resonant capacitor 408. Resonant capacitor 408 may be positioned in series with resonant inductor 410 to define a main resonant circuit, which together create a varying impedance that is minimal at a resonant frequency defined by equation 1 below:

$$Fr = 1/( 2 * pi * sqrt(L*C)). \qquad\qquad (1)$$

The switches 404 and 406 may be made of semiconductor devices (e.g., mosfet, insulated gate bipolar transistor (IGBT), etc.), and may provide a square wave voltage at a controlled frequency with amplitude defined by DC voltage source 402. By controlling the frequency of the resonant circuit via switches 404 and 406, the power delivered to the filament 414 may be controlled. The main resonant circuit can operate in ZeroVoltage Switching (ZVS) or Zero Current Switching (ZCS) modes, which may help minimize switching losses of semiconductor devices and improve converter efficiency.

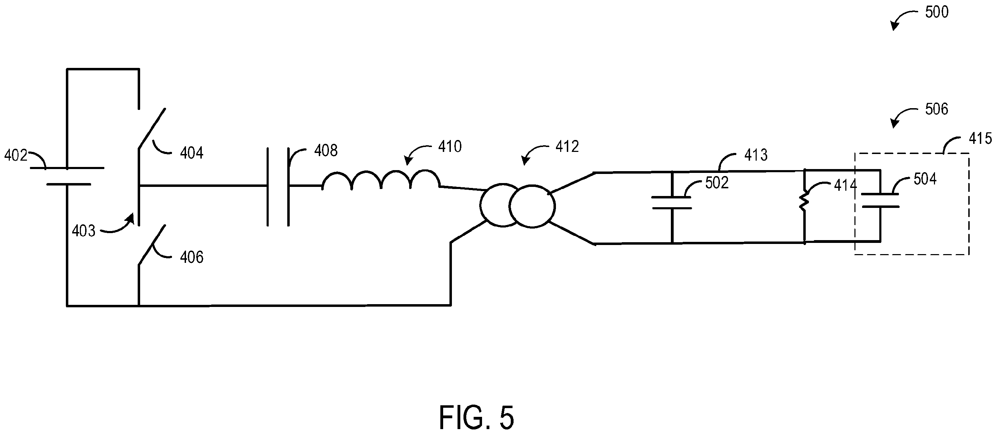

FIG. 5 shows a second exemplary filament drive circuit 500, according to the present disclosure. The filament drive circuit 500 may include similar components as the filament drive circuit 400 and therefore, like components are referred to herein with like reference numerals and may not be reintroduced. In comparison to the filament drive circuit 400, the filament drive circuit 500 additionally comprises a first diagnostic capacitor 502 and a second diagnostic capacitor 504. The first diagnostic capacitor 502 is positioned on the high-voltage cable 413 leading from the high-voltage transformer 412 to the filament 414. The second diagnostic capacitor 504 is positioned at an opposite end 506 of the filament drive circuit 500 (e.g., within the X-ray tube assembly 415), such that the filament 414 is positioned between the second diagnostic capacitor 504 and the first diagnostic capacitor 502. For example, in one embodiment, the second diagnostic capacitor 504 may be located in a high-voltage connector of the X-ray tube assembly 415, where Metal Oxide Varistors (MOVs) may be located.

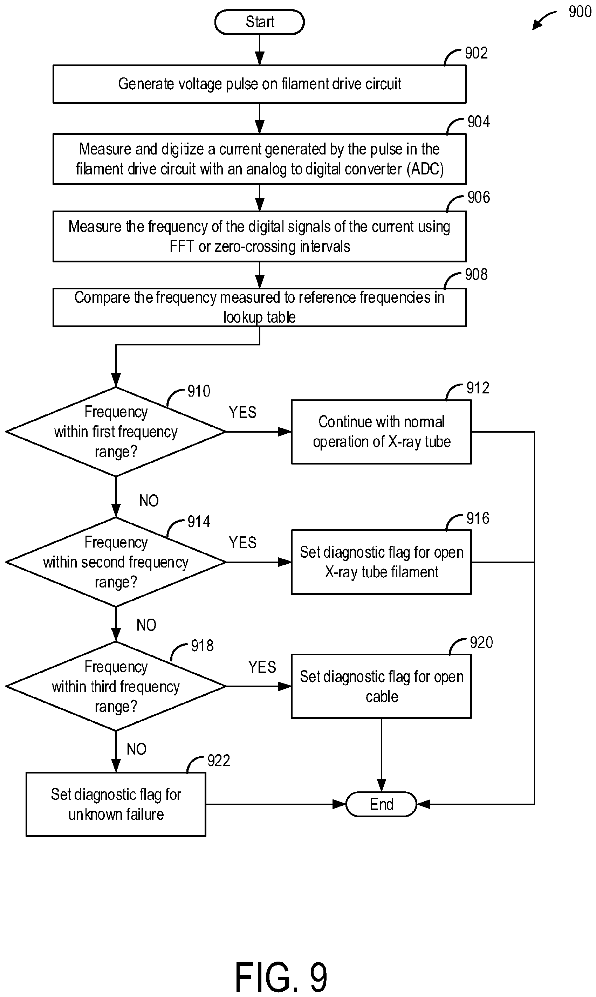

The first diagnostic capacitor 502 and the second diagnostic capacitor 504 may be advantageously used to detect a degradation in the filament 414 and/or other components of filament drive circuit 500, as described in greater detail below in reference to FIG. 9. Specifically, the inclusion of the first diagnostic capacitor 502 and the second diagnostic capacitor 504 may cause the resonant frequency of the filament drive circuit 500 to change in the event that one or more components of the filament drive circuit 500 is degraded.

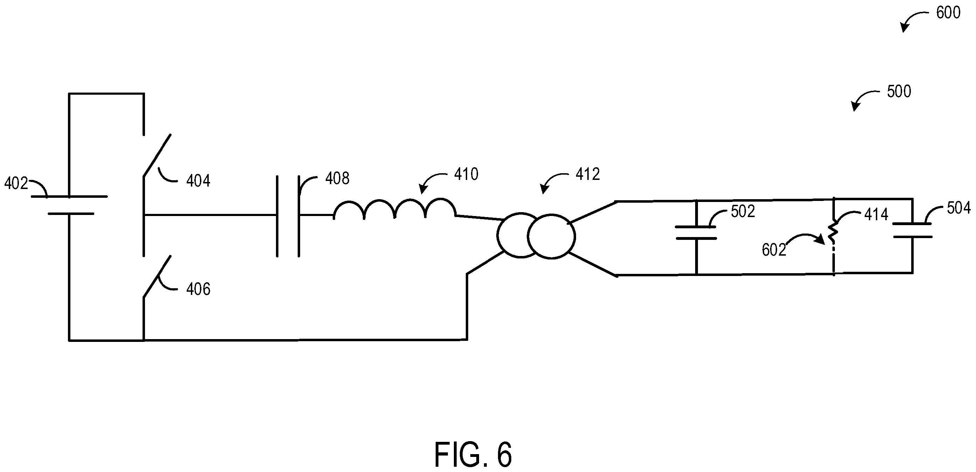

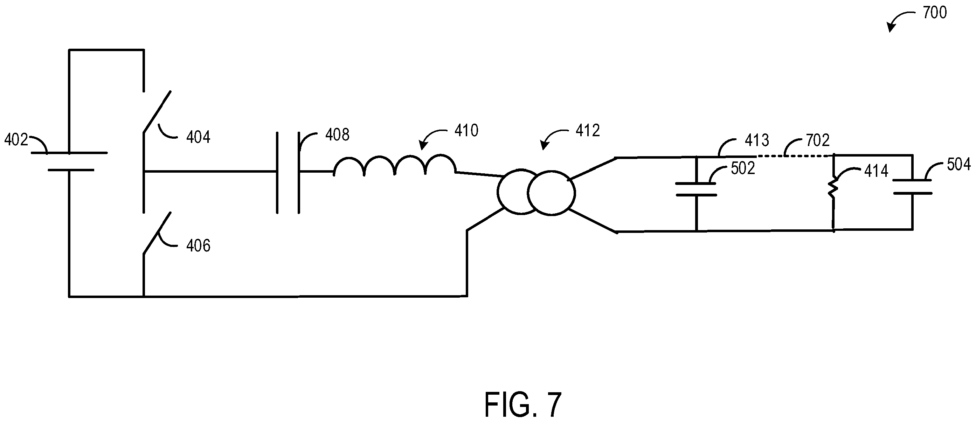

For example, FIGS. 6 and 7 show examples of filament drive circuits of an X-ray imaging system where different components have degraded. FIG. 6 shows a first scenario 600 of the filament drive circuit 500, where the filament 414 is opened at a location 602, and an electric current generated by the voltage source 402 may not pass through filament 414 to complete filament drive circuit 500. For example, the filament 414 may have opened due to a high temperature to which the filament material in the x-ray tube is exposed, which may cause the filament material to slowly evaporate over time. This evaporation process can lead to a decrease in the filament's size, which could lead to an open-circuit type of failure at the end of a lifespan of the x-ray tube filament.

Alternatively, FIG. 7 shows a second scenario 700 of the filament drive circuit 500, where the filament 414 is not opened, but the high-voltage cable 413 has failed at a location 702. For example, the high-voltage cable 413 may have a short circuit, or there may be a defect in the high-voltage cable 413, or there may be a bad connection of the high-voltage cable 413 with a portion of the filament drive circuit 500. As a result of the failure, the electric current generated by the voltage source 402 may not pass through the high-voltage cable 413 to the filament 414. Either of the first scenario 600 and the second scenario 700 may result in an X-ray tube failure.

However, the first scenario 600 may be distinguished from the second scenario 700 by measuring the resonant frequency of the filament drive circuit 500. The measured resonant frequency may be checked against a set of reference resonant frequency measurements. For example, a first reference resonant frequency measurement may correspond to the filament drive circuit 500 where no components are degraded (e.g., normal or expected functioning); a second reference resonant frequency measurement may correspond to the filament drive circuit 500 where the filament 414 is degraded (e.g., the first scenario 600); and a third reference resonant frequency measurement may correspond to the filament drive circuit 500 where the high-voltage cable 413 is degraded (e.g., the second scenario 700).

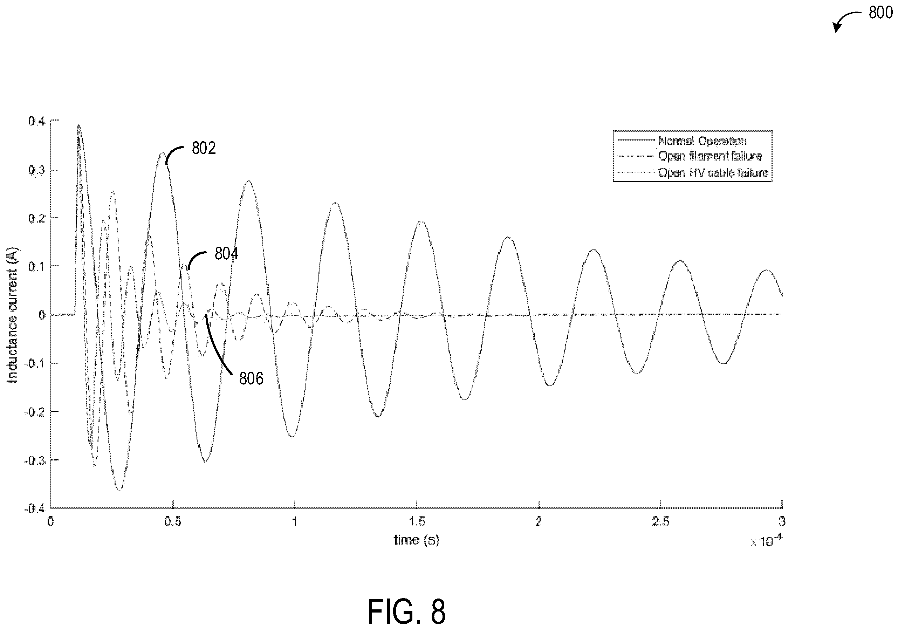

Referring to FIG. 8, a graph 800 depicts an inductance current of the filament drive circuit 500 over time, showing the reference frequencies described above. The first reference resonant frequency is shown by a line 802; the second reference resonant frequency is shown by a line 804; and the third reference resonant frequency is shown by a line 806. A first amplitude of the line 802 is greater than a second amplitude of the line 804, which in turn is greater than a third amplitude of the line 806. Thus, the first, second, and third reference resonant frequencies may be distinguished by their amplitude. As a result, by comparing the measured resonant frequency to each of the first, second, and third reference resonant frequencies, the degraded component may be identified. That is, if the measured resonant frequency matches the first reference resonant frequency, the filament drive circuit 500 may not be diagnosed as having a degraded component; if the measured resonant frequency matches the second reference resonant frequency, the filament drive circuit 500 may be diagnosed as having a degraded filament 414; and if the measured resonant frequency matches the third reference resonant frequency, the filament drive circuit 500 may be diagnosed as having a degraded high-voltage cable 413.

The measured resonant frequency may match a reference frequency of the first, second, or third reference frequencies if a difference between the measured resonant frequency and the reference frequency is less than a threshold difference. For example, if the measured resonant frequency is within a first kHz range, the measured resonant frequency may match the first reference frequency; if the measured resonant frequency is within a second, different kHz range, the measured resonant frequency may match the second reference frequency; if the measured resonant frequency is within a third kHz range, the measured resonant frequency may match the third reference frequency; and so on. Exemplary kHz ranges and thresholds are described in greater detail in reference to FIG. 9 below.

In this way, an accurate diagnosis of a location of a degradation in the filament drive circuit 500 may be provided quickly and efficiently, without relying on a manual troubleshooting or diagnostic process or the inclusion of additional switches. Additionally, unlike the manual troubleshooting or diagnostic process, a diagnostic routine that relies on the disclosed procedure may be performed automatically and/or remotely, saving additional time and effort. For example, the diagnostic routine may be executed by sending an electronic command to a controller of the X-ray system (e.g., controller 210). The electronic command may be initiated by a remote engineer (e.g., an engineer not located at the X-ray system), or the electronic command may be initiated automatically in response to a detection of a failure of the X-ray system. In some embodiments, the electronic command may be initiated when the X-ray system is started up, to ensure a functionality of the X-ray system.

It should be appreciated that while the methods disclosed herein are described with reference to the first scenario 600 and the second scenario 700, the methods may be extended to diagnose other types of failures without departing from the scope of this disclosure. For example, one or more switches (e.g., the switches 404 and 406) may fail, such that the one or more switches are always open or always closed; a current sensor may not be working properly; a transformer (e.g., the high-voltage transformer 412) may fail; or a different component of a filament drive circuit may fail. To distinguish between failures of other components of the filament drive circuit, additional diagnostic capacitors may be positioned at various strategic locations of the filament drive circuit. The additional diagnostic capacitors may adjust the resonant frequency of the filament drive circuit in the manner described above in the event that one of the other components fails, and additional reference resonant frequencies may be included in the set of reference resonant frequencies to diagnose the failures.

Referring now to FIG. 9, an exemplary method 900 is shown for identifying a degradation in a filament drive circuit, such as the filament drive circuit 500 of FIGS. 5, 6, and 7, of an X-ray tube of an X-ray imaging system, such as the X-ray system 100 of FIG. 1 and/or the X-ray imaging system 200 of FIG. 2. Method 900 may be executed by a processor of a controller of the X-ray imaging system, such as a processor of control electronics module 322 of FIG. 3, as part of a diagnostic routine of the X-ray system.

Method 900 begins at 902, where method 900 includes generating a diagnostic voltage pulse on the filament drive circuit. An amplitude of the voltage pulse may be defined by the amplitude of the voltage source (e.g., voltage source 402 of FIG. 5). A duration of the voltage pulse may be selected to avoid having too much current in the filament circuit, and to be at a lower frequency than a standard resonant frequency of the filament drive circuit during operation (e.g., shown by line 802 of FIG. 8). A width of the voltage pulse may be directly controlled the controller by turning switches on and off, such as switches 404 and 406.

At 904, method 900 includes measuring and digitizing a current generated by the voltage pulse in the filament drive circuit with an analog-to-digital converter (ADC). At 906, method 900 further includes measuring the frequency of the digital signals of the current using a fast Fourier transform (FFT) or zero-crossing intervals. A period of the signals may be measured by a processor or similar logic by measuring a number of clock cycles between two zero-crossings of the current waveforms. The duration between the two zero-crossings is equal to the half-period of the resonant current waveform. By measuring several zero-crossing intervals, an accuracy of the frequency measurement may be increased.

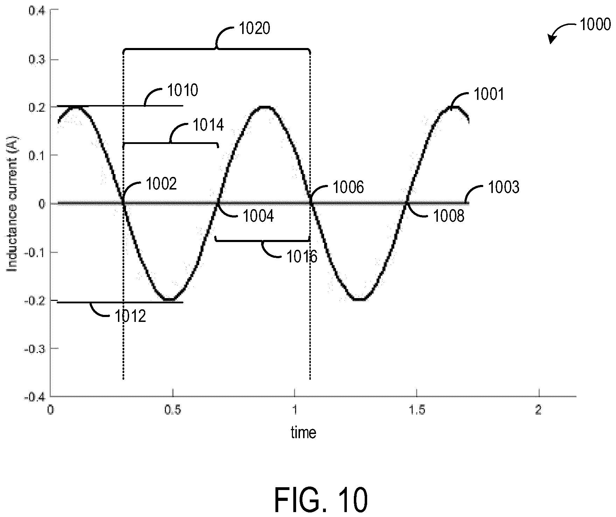

Referring briefly to FIG. 10, a resonant frequency graph 1000 shows an exemplary waveform 1001 of a current generated by a voltage pulse in the filament drive circuit, as described above. The current is plotted on the y axis, and time is plotted on the x axis. Waveform 1001 has an amplitude defined by a distance along the y axis between a first horizontal line 1010 and a second horizontal line 1012, which may be defined by a voltage source (e.g., voltage source 402) of the voltage pulse. Waveform 1001 oscillates between positive and negative current values, such that waveform 1001 crosses a line 1003 indicating a current of 0 a plurality of times. In particular, waveform 1001 crosses line 1003 a first time at a first zero-crossing 1002; a second time at a second zero-crossing 1004; a third time at a third zero-crossing 1006; and a fourth time at a fourth zero-crossing 1002. A resonant frequency of the filament drive circuit is defined by a period 1020 of waveform 1001, where period 1020 may correspond to a distance between first zero-crossing 1002 and third zero-crossing 1006. A first distance 1014 between first zero-crossing 1002 and second zero-crossing 1004 corresponds to a first half-period of waveform 1001, and a second distance 1016 between second zero-crossing 1004 and third zero-crossing 1006 corresponds to a second half-period of waveform 1001. Thus, intervals between various zero-crossings may be measured and averaged to accurately determine the resonant frequency of the filament drive circuit as a result of applying the voltage pulse.

Returning to FIG. 9, at 908, method 900 includes comparing the measured resonant frequency with a set of reference resonant frequencies. In various embodiments, the set of reference resonant frequencies may be stored in a lookup table in a memory of the X-ray imaging system (e.g., storage device 218 of FIG. 2). Each reference resonant frequency of the set of reference resonant frequencies may be caused by a degradation or failure in a different component of the filament drive circuit. Thus, a degradation or failure in a component of the filament drive circuit may be diagnosed by matching the measured resonant frequency with a reference resonant frequency.

At 910, method 900 includes determining whether the frequency is within a first pre-defined frequency range. For example, the first pre-defined frequency range may be between 20-30 kHz. If it is determined that the frequency is within the first pre-defined frequency range, method 900 proceeds to 912. At 912, method 900 includes continuing with normal operation of the X-ray tube. Alternatively, if it is determined that the frequency is not within the first pre-defined frequency range, then method 900 proceeds to 914.

At 914, method 900 includes determining whether the frequency is within a second pre-defined frequency range, where the second pre-defined frequency range is different from the first pre-defined frequency range. The second pre-defined frequency range may be consecutive to the first pre-defined frequency range. For example, the second pre-defined frequency range may be between 30-40 kHz.

If it is determined that the frequency is within the second pre-defined frequency range, method 900 proceeds to 916. At 916, method 900 includes setting a diagnostic flag for an open X-ray tube filament of the filament drive circuit. Alternatively, if it is determined that the frequency is not within the second pre-defined frequency range, then method 900 proceeds to 918.

At 918, method 900 includes determining whether the frequency is within a third pre-defined frequency range, where the third pre-defined frequency range is different from the first pre-defined frequency range and the second pre-defined frequency range. The third pre-defined frequency range may be consecutive to the second pre-defined frequency range. For example, the third pre-defined frequency range may be between 40-50 kHz.

If it is determined that the frequency is within the third pre-defined frequency range, method 900 proceeds to 920. At 920, method 900 includes setting a diagnostic flag for an open high-voltage cable of the filament drive circuit. Alternatively, if it is determined that the frequency is not within the third pre-defined frequency range, then method 900 proceeds to 922.

At 922, method 900 includes setting a diagnostic flag for an unknown failure, and method 900 ends.

Thus, an efficient and inexpensive method for determining where a failure occurred in a filament drive circuit of an X-ray system is disclosed, based on including diagnostic capacitors at strategic locations within the filament drive circuit. Because the diagnostic capacitors may alter a resonant frequency of the filament drive circuit in different ways in the event of a failure of different components of the filament drive circuit, a resonant frequency measured after a failure may be matched with a reference resonant frequency to determine a source of the failure. In contrast with current, manual diagnostic procedures that rely on an engineer testing an impedance of the filament drive circuit at various locations onsite, the method disclosed herein may be performed remotely with limited human intervention. The method may also be performed frequently or routinely with little additional cost. For example, unlike current methods that consume more time and effort, the X-ray system may be tested daily at device startup. As a result, the X-ray system may be maintained in a functional state with less human intervention and at a lower cost using the method than with other diagnostic methods. When a component fails, the failed component may be rapidly and efficiently identified and replaced, without ordering other components that may not be used. The technical effect of detecting a failed component of a filament drive circuit by matching a measured resonant frequency of the filament drive circuit caused by including a diagnostic capacitor in the filament drive circuit with a reference resonant frequency is that the failed component may be detected more rapidly, efficiently, and with less human intervention than current diagnostic methods.

The disclosure also provides support for an X-ray system, comprising: an X-ray tube including a filament, and a filament drive circuit configured to supply a first current from a voltage source to the filament, the filament drive circuit including a high-voltage transformer, an inverter, a resonant inductor, and one or more diagnostic capacitors, wherein each diagnostic capacitor of the one or more diagnostic capacitors corresponds to a component of the filament drive circuit. In a first example of the system, the system further comprises: a controller including a processor and a memory storing instructions that when executed, cause the processor to: generate a diagnostic voltage pulse via the inverter, measure a resonant frequency of the filament drive circuit resulting from the diagnostic voltage pulse, and identify a degradation condition of the X-ray system based on the measured resonant frequency. In a second example of the system, optionally including the first example, identifying the degradation condition of the X-ray system based on the measured resonant frequency further comprises comparing the measured resonant frequency to a set of reference resonant frequencies, and in response to a difference between the measured resonant frequency and a reference resonant frequency of the set of reference resonant frequencies being less than a threshold difference, identifying a degraded component of the filament drive circuit based on the reference resonant frequency. In a third example of the system, optionally including one or both of the first and second examples, each reference resonant frequency of the set of reference resonant frequencies is generated as a result of a degradation of a different component of the filament drive circuit, based on a diagnostic capacitor corresponding to the different component. In a fourth example of the system, optionally including one or more or each of the first through third examples, the set of reference resonant frequencies is stored in a lookup table in the memory of the controller. In a fifth example of the system, optionally including one or more or each of the first through fourth examples, the degraded component of the filament drive circuit is one of: the filament, the high-voltage transformer, the inverter, and a high-voltage cable. In a sixth example of the system, optionally including one or more or each of the first through fifth examples,. in response to the measured resonant frequency being within a first frequency range, no degraded components are identified, in response to the measured resonant frequency being within a second frequency range, a degradation in the X-ray tube is identified, and in response to the measured resonant frequency being within a third frequency range, a degradation in the high-voltage cable is identified, wherein the first frequency range, the second frequency range, and the third frequency range are different frequency ranges. In a seventh example of the system, optionally including one or more or each of the first through sixth examples, the diagnostic voltage pulse is generated in response to a command initiated automatically in response to a detection of a failure of the X-ray system. In an eighth example of the system, optionally including one or more or each of the first through seventh examples, the system further comprises: generating the diagnostic voltage pulse, measuring the resonant frequency of the filament drive circuit, and identifying the degradation condition within a diagnostic routine that is performed on the X-ray system by a remote engineer not located at the X-ray system. In a ninth example of the system, optionally including one or more or each of the first through eighth examples, measuring the resonant frequency of the filament drive circuit further comprises: digitizing a second current generated by the voltage pulse using an analog-to-digital converter, and measuring a frequency of digital signals of the second current using a fast Fourier transform (FFT) or zero-crossing intervals. In a tenth example of the system, optionally including one or more or each of the first through ninth examples, the degradation condition of the X-ray system is identified without checking an impedance at a location in the filament drive circuit.

The disclosure also provides support for a diagnostic routine for an X-ray system, the diagnostic routine comprising: generating a voltage pulse on a filament drive circuit of an X-ray tube of the X-ray system, measuring a resonant frequency of the filament drive circuit resulting from the voltage pulse, and identifying a failed component of the X-ray system based on the measured resonant frequency. In a first example of the system, the filament drive circuit includes a diagnostic capacitor that changes the resonant frequency of the filament drive circuit when a component of the X-ray system corresponding to the diagnostic capacitor fails. In a second example of the system, optionally including the first example, measuring the resonant frequency of the filament drive circuit resulting from the voltage pulse further comprises: digitizing a current generated by the voltage pulse using an analog-to-digital converter, and measuring a frequency of digital signals of the current using a fast Fourier transform (FFT) or zero-crossing intervals. In a third example of the system, optionally including one or both of the first and second examples, identifying the failed component of the X-ray system based on the measured resonant frequency further comprises: comparing the measured resonant frequency to a set of reference resonant frequencies stored in a memory of the X-ray system, and in response to a difference between the measured resonant frequency and a reference resonant frequency of the set of reference resonant frequencies being less than a threshold difference, identifying the failed component based on the reference resonant frequency. In a fourth example of the system, optionally including one or more or each of the first through third examples, in response to the measured resonant frequency being within a first frequency range, no failed components are identified, in response to the measured resonant frequency being within a second frequency range, the failed component is identified as the filament, and in response to the measured resonant frequency being within a third frequency range, the failed component is identified as a high-voltage cable of the X-ray system, wherein the first frequency range, the second frequency range, and the third frequency range are different frequency ranges. In a fifth example of the system, optionally including one or more or each of the first through fourth examples, the diagnostic routine is executed by sending an electronic command to a controller of the X-ray system, the electronic command initiated automatically when the X-ray system is started up and in response to a detection of a failure of the X-ray system.

The disclosure also provides support for an X-ray system, comprising: an X-ray tube, a filament drive circuit configured to supply a current to a filament of the X-ray tube, a first diagnostic capacitor positioned at a first location within the filament drive circuit, a second diagnostic capacitor positioned at a second location within the filament drive circuit, and a processor and a memory including instructions that when executed, cause the processor to: generate a voltage pulse on the filament drive circuit, in response to the filament drive circuit resonating at a first resonant frequency caused by the first diagnostic capacitor, set a first diagnostic flag of the X-ray system indicating a failure of a first component of the filament drive circuit, and in response to the filament drive circuit resonating at a second resonant frequency caused by the second diagnostic capacitor, the second resonant frequency different from the first resonant frequency, set a diagnostic flag of the X-ray system indicating a failure of a second component of the filament drive circuit. In a first example of the system, the first resonant frequency is a frequency within a first pre-defined frequency range stored in a memory of the X-ray system, and the second resonant frequency is a frequency within a second pre-defined frequency range stored in a memory of the X-ray system. In a second example of the system, optionally including the first example, the first component and the second component are each one of: a filament of an X-ray tube of the X-ray system, a voltage source, a high-voltage transformer, an inverter, and a resonant inductor.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative only and should not be construed to be limiting in any manner.

**Claims**

1. An X-ray system (100, 200), comprising:

   an X-ray tube (300) including a filament (414, 308); and
   a filament drive circuit (500) configured to supply a first current from a voltage source (402, 310) to the filament (414, 308), the filament drive circuit (500) including a high-voltage transformer (412), an inverter (403), a resonant inductor (410), and one or more diagnostic capacitors (502, 504);
   wherein each diagnostic capacitor (502, 504) of the one or more diagnostic capacitors (502, 504) corresponds to a component of the filament drive circuit (500).

2. The X-ray system (100, 200) of claim 1, further comprising a controller (210) including a processor and a memory storing instructions that when executed, cause the processor to:

   generate a diagnostic voltage pulse via the inverter (403);
   measure a resonant frequency of the filament drive circuit (500) resulting from the diagnostic voltage pulse; and
   identify a degradation condition of the X-ray system (100, 200) based on the measured resonant frequency.

3. The X-ray system (100, 200) of claim 2, wherein identifying the degradation condition of the X-ray system (100, 200) based on the measured resonant frequency further comprises comparing the measured resonant frequency to a set of reference resonant frequencies, and in response to a difference between the measured resonant frequency and a reference resonant frequency of the set of reference resonant frequencies being less than a threshold difference, identifying a degraded component of the filament drive circuit (500) based on the reference resonant frequency.

4. The X-ray system (100, 200) of claim 3, wherein each reference resonant frequency of the set of reference resonant frequencies is generated as a result of a degradation of a different component of the filament drive circuit (500), based on a diagnostic capacitor (502, 504) corresponding to the different component.

5. The X-ray system (100, 200) of claim 3, wherein the set of reference resonant frequencies is stored in a lookup table (114) in the memory of the controller (210).

6. The X-ray system (100, 200) of claim 3, wherein the degraded component of the filament drive circuit (500) is one of:

    the filament (414);
    the high-voltage transformer (412);
    the inverter (403); and
    a high-voltage cable (413).

7. The X-ray system (100, 200) of claim 6, wherein:

    in response to the measured resonant frequency being within a first frequency range, no degraded components are identified;
    in response to the measured resonant frequency being within a second frequency range, a degradation in the filament (414, 308) is identified; and
    in response to the measured resonant frequency being within a third frequency range, a degradation in the high-voltage cable (413) is identified;
    wherein the first frequency range, the second frequency range, and the third frequency range are different frequency ranges.

8. The X-ray system (100, 200) of claim 2, further comprising generating the diagnostic voltage pulse in response to a command initiated automatically in response to a detection of a failure of the X-ray system (100, 200).

9. The X-ray system (100, 200) of claim 2, further comprising generating the diagnostic voltage pulse, measuring the resonant frequency of the filament drive circuit (500), and identifying the degradation condition within a diagnostic routine that is performed on the X-ray system (100, 200) by a remote engineer not located at the X-ray system (100, 200).

10. The X-ray system (100, 200) of claim 2, wherein measuring the resonant frequency of the filament drive circuit (500) further comprises:

    digitizing a second current generated by the voltage pulse using an analog-to-digital converter; and
    measuring a frequency of digital signals of the second current using a fast Fourier transform (FFT) or zero-crossing intervals.

11. The X-ray system (100, 200) of claim 2, wherein the degradation condition of the X-ray system (100, 200) is identified without checking an impedance at a location (602, 702) in the filament drive circuit (500).

12. A diagnostic routine for an X-ray system, the diagnostic routine comprising:

    generating a voltage pulse on a filament drive circuit of an X-ray tube of the X-ray system (902);
    measuring a resonant frequency of the filament drive circuit resulting from the voltage pulse (906); and
    identifying a failed component of the X-ray system based on the measured resonant frequency (916, 920).

13. The diagnostic routine of claim 12, wherein the filament drive circuit includes a diagnostic capacitor that changes the resonant frequency of the filament drive circuit when a component of the X-ray system corresponding to the diagnostic capacitor fails.

14. The diagnostic routine of claim 12, wherein measuring the resonant frequency of the filament drive circuit resulting from the voltage pulse further comprises:

digitizing a current generated by the voltage pulse using an analog-to-digital converter (904); and
measuring a frequency of digital signals of the current using a fast Fourier transform (FFT) or zero-crossing intervals (906).

15. The diagnostic routine of claim 12, wherein identifying the failed component of the X-ray system based on the measured resonant frequency further comprises:

comparing the measured resonant frequency to a set of reference resonant frequencies stored in a memory of the X-ray system (908); and
in response to a difference between the measured resonant frequency and a reference resonant frequency of the set of reference resonant frequencies being less than a threshold difference, identifying the failed component based on the reference resonant frequency (916, 920).

FIG. 1

EP 4 622 396 A1

FIG. 2

FIG. 3

FIG. 4 (Prior Art)

EP 4 622 396 A1

FIG. 5

600

500

504

414

602

502

412

410

408

404

406

402

FIG. 6

FIG. 7

EP 4 622 396 A1

FIG. 8

EP 4 622 396 A1

900

Start

Generate voltage pulse on filament drive circuit ⌐902

Measure and digitize a current generated by the pulse in the filament drive circuit with an analog to digital converter (ADC) ⌐904

Measure the frequency of the digital signals of the current using FFT or zero-crossing intervals ⌐906

Compare the frequency measured to reference frequencies in lookup table ⌐908

Frequency within first frequency range? ⌐910 — YES → Continue with normal operation of X-ray tube ⌐912

NO

Frequency within second frequency range? ⌐914 — YES → Set diagnostic flag for open X-ray tube filament ⌐916

NO

Frequency within third frequency range? ⌐918 — YES → Set diagnostic flag for open cable ⌐920

NO

Set diagnostic flag for unknown failure ⌐922

End

FIG. 9

FIG. 10

EP 4 622 396 A1

Europäisches
Patentamt

European
Patent Office

Office européen
des brevets

# EUROPEAN SEARCH REPORT

**Application Number**

EP 25 15 9597

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/009918 A1 (BELAND ROBERT [CA]) 8 January 2009 (2009-01-08) * see fig. 8 and the description thereof; * | 1 | INV. H05G1/10 H05G1/54 H05G1/22 |
| A | SU 1 363 543 A1 (NII INTROSKOPII [SU]) 30 December 1987 (1987-12-30) * the whole document * | 1,12 | |

**TECHNICAL FIELDS SEARCHED (IPC)**

H05G

The present search report has been drawn up for all claims

| Place of search | Date of completion of the search | Examiner |
|---|---|---|
| Munich | 31 July 2025 | Angloher, Godehard |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 15 9597

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2025

| Patent document cited in search report | Publication date | Patent family member(s) | Publication date |
|---|---|---|---|
| US 2009009918 A1 | 08-01-2009 | US 6738275 B1<br>US 2004264222 A1<br>US 2006187691 A1<br>US 2009009918 A1<br>US 2011286580 A1 | 18-05-2004<br>30-12-2004<br>24-08-2006<br>08-01-2009<br>24-11-2011 |
| SU 1363543 A1 | 30-12-1987 | NONE | |

EPO FORM P0459

For more details about this annex : see Official Journal of the European Patent Office, No. 12/82

**EP 4 622 396 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202441020353